# EUROPEAN PATENT APPLICATION

(11) **EP 4 193 999 A1**
(43) Date of publication of application: **14.06.2023**
(21) Application number: 21855503.5
(22) Date of filing: 10.08.2021
(51) Int. Cl.: A61K 35/28, A61K 45/00, A61P 11/00

(54) **USES OF MSC IN IMPROVING THROMBOTIC COMPLICATIONS IN COVID-19 PNEUMONIA**

(30) Priority: 10.08.2020 CN 202010794338
(71) Applicant: Shanghai University, Shanghai 200444 (CN)
(72) Inventor: ZHAO, Chunhua, Shanghai 200444 (CN)
(74) Representative: Sonzogni, Laura Gabriella
(86) International application number: PCT/CN2021/111649
(87) International publication number: WO 2022/033451

(57) **Abstract**

Use of an MSC in improving thrombotic complications in COVID-19 pneumonia. Provided is an ACE2-negative MSC; an intravenous injection of the MSC is safe and efficacious for critical and severe COVID-19 patients. The ACE2-negative MSC promotes organ injury repairing by means of immunomodulation. Also discovered is that the MSC is capable of upregulating the expression of kindlin-3 in immune cells, thus upregulating integrin signaling in the immune cells, and inhibiting the release of neutrophil extracellular traps (NETs) in a COVID-19 patient. This favors an improvement on thrombotic complications in COVID-19 pneumonia.

## Description

The present application claims the priority of Chinese patent application No. 202010794338.4 filed on August 10, 2020.

### FIELD OF THE INVENTION

The present disclosure belongs to the technical field of biomedicine and relates to ACE2-negative mesenchymal stem cells (hereinafter referred to as MSCs) for use in treating thrombotic complications of severe and critical COVID-19 pneumonia. Especially, the MSCs can improve thrombotic complications and reduce the incidence of thrombotic complications by inhibiting the release of neutrophil extracellular traps.

### BACKGROUND OF THE INVENTION

COVID-19 is an infectious disease caused by SARS-CoV-2. The disease is characterized by fever, cough, fatigue, diarrhea, pneumonia, thrombosis, as well as some neurological and psychotic symptoms.

Approximately 20% of COVID-19 patients develop severe diseases (Wu et al., 2020). Although the pathogenesis of the SARS-CoV-2 virus remains unclear, dysfunction of the human immune system and inflammatory cytokine storms as well as inflammatory cell infiltration in tissue are thought to be associated with the severity of the disease. In the COVID-19 epidemic, studies have also found a decrease in the number of peripheral blood lymphocytes and an increase in serum inflammatory cytokine levels (Huang et al., 2020; Wang et al., 2020; Chen et al., 2020). Fa Klok et al., 2020 (published in Thromb Res) aimed to observe the incidence of thrombotic complications in patients with severe COVID-19 in ICU, and reported an incidence of thrombotic complications as high as 31% in patients with COVID-19 infections in the ICU. This finding underscores the importance of thromboprophylaxis for all COVID-19 patients in the ICU.

MSCs, also known as pluripotent mesenchymal stromal cells, are heterogeneous populations (Uccelli et al., 2008). The discovery of MSCs is based on the observation that human bone marrow (BM) cell suspensions cultured in petri dishes lose their hematopoietic components, favoring the proliferation and adhesion of fibroblast-like cells and thus the formation of colonies, which can differentiate into adipocytes, chondrocytes and osteocytes *in vitro* (Friedenstein et al., 1968). Pittenger et al., 1999 demonstrated the multilineage differentiation ability of MSCs. Numerous studies have explored the role of MSCs in tissue repair and the regulation of allogeneic immune responses. The mechanism by which MSCs realize their therapeutic potential depends on some key properties of the cells, as follows:
- the ability of secreting a variety of biologically active molecules, which are able to stimulate the recovery of injured cells and suppress inflammation; and
- the ability of performing immunomodulatory functions.

In clinical trials registered in clinical trial databases (e.g., http://clinicaltrials.gov), MSCs are used to treat multiple immune diseases such as graft-versus-host disease (GVHD), systemic lupus erythematosus and multiple sclerosis.

Neutrophil extracellular traps (NETs) are extracellular reticular structures composed of chromatin fiber and microbicidal granule components. NET levels significantly increased in plasma of patients with COVID-19-associated acute respiratory distress syndrome. However, it remains unclear whether MSCs can inhibit NET release in COVID-19 patients.

### SUMMARY OF THE INVENTION

MSCs play a regulatory role in immunity and have the ability of tissue repair. MSCs have become an attractive therapeutic cell type for acute/chronic and severe immune diseases.

The inventors have unexpectedly discovered that MSCs do not express ACE2 receptors and therefore are not the targets for SARS-CoV-2 and thus resistant to SARS-CoV-2 infection.

In the present disclosure, the inventors have found that ACE2-negative MSCs can improve thrombotic complications of severe and critical COVID-19 pneumonia, effectively promote the prognosis of patients with severe and critical COVID-19.

### ACE2-negative MSCs

According to some embodiments of the present disclosure, provided are MSCs.

According to some particular embodiments of the present disclosure, provided are ACE2-negative MSCs.

In the present application, "ACE2-negative" (also referred to as ACE2⁻) means that the ACE2 level in the MSCs or on their cell surface is undetectable using the detection method for ACE2 well-known in the prior art.

In the present application, ACE2 refers to mammal (specifically human) angiotensin-converting enzyme 2. ACE2 should be understood in the broadest way and include any form of ACE2, for example, but not limited to, ACE2 in its native state, in a transmembrane form, naturally occurring variants, or fragments thereof. SARS-CoV-2 enters cells by recognizing ACE2 of the host (mammal, specifically human).

MSCs have the ability of self-regeneration and are able to differentiate into multiple cell lineages of mesenchymal tissue.

In some embodiments, the MSCs are derived from adipose, umbilical cord blood, umbilical cord, bone marrow or placenta.

In other embodiments, the MSCs are autologous MSCs or allogeneic MSCs.

In some embodiments, the MSCs are clinical grade MSCs.

### Culture methods for MSCs

The present application relates to a method of treating MSCs, the MSCs are preferably derived from adipose tissue (obtained and/or isolated from adipose tissue of adult animals); more specifically from animal sources, preferably human. This method mainly requires two steps: 1) obtaining and/or isolating MSCs; 2) growing and/or treating MSCs in culture medium for a period of time.

In one embodiment, the MSCs are isolated or purified from bone marrow. In another embodiment, the MSCs are bone marrow-derived MSCs. In another embodiment, the MSCs are isolated or purified from adipose tissue. In another embodiment, the MSCs are isolated or purified from cartilage. In another embodiment, the MSCs are isolated or purified from any other tissue known in the art.

In some embodiments, adipose tissue-derived MSCs can be isolated from human tissues according to the methods described in Yoshimura et al., 2006; Almeida et al., 2008; Wagner et al., 2005. For example, adipose tissue-derived MSCs are obtained from adipose tissue in anaesthetized healthy patients. The adipose tissue was washed with PBS, digested with type I collagenase at 37°C, and centrifuged to obtain cell aggregates. The cell aggregates were suspended in erythrocyte lysis buffer. The cell suspension was filtered through a filter and centrifuged. After suspension of the cells, they were seeded in an appropriate culture medium for cell expansion.

Any method, step, parameter and condition applicable to clinical grade MSC culture in the art is applicable to the present application. The criteria for the identification of clinical grade MSCs are well known. For example, Quality Control and Technical Specifications of Clinical Grade Mesenchymal Stem Cells from Human Tissues" (Industry standard DB32T: 3544-2019); Chen Jin et al., Specification for culture of clinical grade mesenchymal stem cells, Chinese Journal of Cell and Stem Cells, 2013, 003(003): 27-31; Huaijuan Ren, Research on the library construction and preparation process for clinical grade mesenchymal stem cells, Shanghai Jiao Tong University, 2016.

In some embodiments, before being administered to the patient, ACE2-negative MSCs are obtained by any of the following culture conditions or a combination thereof:
at a temperature of 30°C to 40°C, for example 30, 31, 32, 33, 34, 35, 36, 37, 38, 39, 40°C, and the range between any two values (including non-integer values);
in an atmosphere of 4% to 6% of CO₂ (for example 4.1, 4.2, 4.3, 4.4, 4.5, 4.6, 4.7, 4.8, 4.9, 5.0, 5.1, 5.2, 5.3, 5.4, 5.5, 5.6, 5.7, 5.8, 5.9, 6.0% of CO₂);
culturing MSCs in DMEM/F12 medium for 2 to 20 passages (for example for 1, 2, 3, 4, 5, 6, 7, 8, 9, 10 passages).

In some embodiments, the DMEM/F12 medium is supplemented with any one selected from the following or a combination thereof:
0.1% to 30% w/v (for example 0.1, 0.5, 1, 2, 3, 4, 5, 6, 7, 8, 9, 10, 15, 20, 25% w/v) of FBS,
1% to 3% w/v (for example 1, 1.5, 2, 2.5, 3% w/v) of antibiotics (for example penicillin/streptomycin), and
0.1 mM to 30 mM (for example 0.1, 0.5, 1, 2, 3, 4, 5, 6, 7, 8, 9, 10, 15, 20, 25 mM) of GlutaMAX^{™}-I.

### Uses and treatment methods of MSCs

According to some embodiments of the present application, also provided is use of the aforementioned ACE2-negative MSCs in treating viral pneumonia.

According to other embodiments of the present application, also provided is use of the aforementioned ACE2-negative MSCs in preparing a medicament for treating viral pneumonia.

In some particular embodiments, the ACE2-negative MSCs of the present application are especially effective for severe or critical viral pneumonia.

In some particular embodiments, the ACE2-negative MSCs of the present application are used to treat thrombotic complications of severe or critical viral pneumonia.

Thrombus is a clot that forms on the exfoliated or repaired surface of the inner surface of a blood vessel. One of the causes of blood clots is an autoimmune antibody that circulates in the blood and attacks cells to trigger clot formation in arteries, veins and capillaries. Blood clots can cause life-threatening symptoms, such as stroke. Thrombosis is one of the severe complications that occur in COVID-19 patients, and the clots restrict blood flow in lungs, thus affecting oxygen exchange.

In some particular embodiments, the ACE2-negative MSCs of the present application is used for any one selected from the following: improving the symptoms of thrombotic complications of viral pneumonia, reducing the incidence of thrombotic complications of viral pneumonia, improving the prognosis of thrombotic complications of viral pneumonia, and inhibiting the release of neutrophil extracellular traps.

In some particular embodiments, treatment of viral pneumonia by ACE2-negative MSCs is embodied in any one selected from the following: improving the symptoms of thrombotic complications of viral pneumonia, reducing the incidence of thrombotic complications of viral pneumonia, improving the prognosis of thrombotic complications of viral pneumonia, and inhibiting the release of neutrophil extracellular traps.

In some particular embodiments, the virus is selected from the group consisting of rhinovirus, coronavirus, adenovirus, influenza virus, parainfluenza virus, respiratory syncytial virus, echovirus, coxsackie virus and variants thereof, wherein the coronavirus is selected from the group consisting of SARS-CoV, MERS-CoV, 2019-nCoV and variants thereof.

According to other embodiments of the present application, also provided is a method of treating thrombotic complications of viral pneumonia, including a step of administering a therapeutically effective amount of ACE2-negative MSCs to a patient.

In some embodiments of the method according to the present application, the patient is a carrier of the virus, especially a patient who has or may have symptoms due to the presence of the virus. The virus is selected from the group consisting of rhinovirus, coronavirus (mention may be made of SARS-CoV, MERS-CoV and 2019-nCoV), adenovirus, influenza virus, parainfluenza virus, respiratory syncytial virus, echovirus and coxsackie virus.

In particular embodiments, the patient is especially a patient with severe or critical disease.

In some embodiments of the method according to the present application, the MSCs are selected from the group consisting of adipose MSCs, umbilical cord blood MSCs, umbilical cord MSCs, bone marrow MSCs, placenta MSCs and dental pulp MSCs. In some embodiments of the method according to the present application, the MSCs are autologous MSCs or allogeneic MSCs.

In some embodiments of the method according to the present application, a "therapeutically effective amount" or "effective dose" refers to an amount of a medicament, compound or pharmaceutical composition necessary to obtain any one or more beneficial or desired therapeutic results. The beneficial or desired results include improving clinical outcomes (such as reducing morbidity and mortality, improving one or more symptoms), reducing severity, delaying the onset of the condition (including the condition or complications thereof, intermediate pathological phenotypes that appear during the development of the condition, biochemistry, histology and/or behavioral symptoms).

In some embodiments, the effective amount of treatment is 0.1×10⁵ to 9×10⁶ MSCs per kilogram of body weight, and mention may be made of 0.1×10⁵, 0.2×10⁵, 0.3×10⁵, 0.4×10⁵, 0.5×10⁵, 0.6×10⁵, 0.7×10⁵, 0.8×10⁵, 0.9×10⁵, 1×10⁵, 1.5×10⁵, 2×10⁵, 2.5×10⁵, 3×10⁵, 3.5×10⁵, 4×10⁵ , 4.5×10⁵, 5×10⁵, 5.5×10⁵, 6×10⁵, 6.5×10⁵, 7×10⁵, 7.5×10⁵, 8×10⁵, 8.5×10⁵, 9×10⁵, 9.5×10⁵, 1×10⁶, 1.5×10⁶, 2×10⁶, 2.5×10⁶, 3×10⁶, 3.5×10⁶, 4×10⁶, 4.5×10⁶, 5×10⁶, 5.5×10⁶, 6×10⁶, 6.5×10⁶, 7×10⁶, 7.5×10⁶, 8×10⁶, 8.5×10⁶, 9×10⁶, or the ranges between any two of the above values. In some particular embodiments, the therapeutically effective amount is 1×10⁶ MSCs per kilogram of body weight.

### NET release inhibitors

In some embodiments, provided is a NET release inhibitor.

NET is a microbicidal mechanism of neutrophils. NETs are composed of nucleic acid substances and do not contain cytoskeletal proteins. The nucleic acid substances include DNA and granule proteins. DNA is the main part of NETs and forms a skeleton that holds various protein granules. It can be observed under high-resolution scanning electron microscopy that granule proteins include primary granules from neutrophils (composed of elastase, cathepsin G, myeloperoxidase, etc.), secondary granules (composed of lactoferrin, gelatinase, etc.) and tertiary granules.

NET release inhibitor refers to any active substance that has the ability of inhibiting NETs, the inhibition is selected from any aspect of the following or a combination thereof: inhibiting NET formation, inhibiting NET release, reducing effective levels of NETs, inhibiting NET activity/function, and blocking NET-related pathways.

In some embodiments, the NET release inhibitor is selected from the group consisting of kindlin-3, an agent that promotes the expression of kindlin-3, a cell that expresses kindlin-3 on its surface, and a viral vector that expresses kindlin-3. The kindlin family is a family of vinculins, and three members, kindlin-1, -2 and -3, have been identified. Mammal kindlin also has a typical FERM domain (composed of F1, F2 and F3 subdomains) that can interact with the extracellular segment of transmembrane proteins.

In the present application, kindlin-3 refers to mammal (specifically human) kindlin-3. The kindlin-3 should be understood in the broadest way to include any form of kindlin-3, for example but not limited to the different forms of kindlin-3 at any stage of the expression process, such as kindlin-3 in its native state, precursors of kindlin-3, mature proteins of kindlin-3, naturally occurring variants, or fragments thereof.

Promoting the expression of kindlin-3 refers to increasing the expression level or amount of kindlin-3 in patients (especially in immune cells), or prolonging the half-life of kindlin-3 in patients (especially in immune cells), or improving the activity of kindlin-3 in patients (especially in immune cells).

An agent that promotes the expression of kindlin-3 refers to any active substance that has the ability of promoting the expression of kindlin-3. In one particular embodiment, the agent that promotes the expression of kindlin-3 is the ACE2-negative mesenchymal stem cells defined above.

According to some embodiments, provided is use of a NET release inhibitor in the manufacture a medicament for treating viral pneumonia. In some embodiments, the NET release inhibitor is used for any one selected from following: improving the symptoms of thrombotic complications of viral pneumonia, reducing the incidence of thrombotic complications of viral pneumonia, and improving the prognosis of thrombotic complications of viral pneumonia. In some embodiments, the viral pneumonia is severe viral pneumonia or critical viral pneumonia. In some embodiments, the virus is selected from the group consisting of SARS-CoV, MERS-CoV, 2019-nCoV and variants thereof.

According to some embodiments, provided is a method of treating thrombotic complications of viral pneumonia, including a step of administering a therapeutically effective amount of a NET release inhibitor to a patient, wherein the NET release inhibitor is used for any one selected from the following: improving the symptoms of thrombotic complications of viral pneumonia, reducing the incidence of thrombotic complications of viral pneumonia, and improving the prognosis of thrombotic complications of viral pneumonia. In some embodiments, the NET release inhibitor is selected from the group consisting of kindlin-3, an agent that promotes the expression of kindlin-3, a cell that expresses kindlin-3 on its surface, and a viral vector that expresses kindlin-3. In some embodiments, the viral pneumonia is severe viral pneumonia or critical viral pneumonia. In some embodiments, the virus is selected from the group consisting of SARS-CoV, MERS-CoV, 2019-nCoV and variants thereof.

### DESCRIPTION OF THE DRAWINGS

Figure 1A: Cumulative symptom relief rates of the MSC-treated group and placebo group.
Figure 1B: CRP levels in the MSC-treated group and placebo group.
Figure 2A to Figure 2L: Ratio of the mean of each cytokine (on Day 28 after MSC or placebo infusion) to the mean at baseline (before treatment).
Figure 3A: Plasma NET-DNA levels of MSC-treated patients at three time points (n = 29, p = 0.01, Day 7.5±1.5 versus Day 0).
Figure 3B: Change in NET-DNA levels in the plasma of MSC-treated patients over time (n = 22, p = 0.0483, data on Day 7 and Day 0).
Figure 3C: Change in plasma NET-DNA levels in placebo-treated patients over time (n = 7, p > 0.05).
Figure 4A: Antibodies against the SARS-CoV-2 S1+S2 extracellular domain, receptor-binding domain, or nucleocapsid detected in the plasma of healthy subjects and placebo-treated patients.
Figure 4B: Three specific antibodies (specific for S1+S2, RBD, and N epitope) were detected in plasma samples from the MSC-treated group and placebo group (p > 0.05).
Figure 5: Ratio of antibody levels on Day 28 to those on Day 14 in the MSC-treated group and placebo group. Data represent mean ± SD. P values were determined by unpaired Student's t-test (* p < 0.05; ** p < 0.01; *** p < 0.001).
Figure 6A to Figure 6C: Results of differential expression profiling by single-cell RNA sequencing. Gene expression of integrin β2 subunit, Talin and kindlin-3 in innate immune cells and lymphocytes in MSC-treated patients was upregulated compared to untreated patients.
Figure 7: Soluble DNA levels in the plasma of MSC-treated COVID-19 patients were significantly lower than that in untreated patients. MSC-D4 refers to Day 4 after MSC infusion.
Figure 8: Significant increase in circulating DNA levels in the plasma of both types of mice caused by IVC stenosis.
Figure 9 and Figure 10: Thrombosis was significantly hindered in mice expressing EGFP-kindlin-3 (EGFP-K3) compared to that in mice expressing EGFP alone.

### DETAILED DESCRIPTION OF THE INVENTION

### Example 1. Inclusion and exclusion criteria of patients

### 1. All patients were diagnosed by 2019-nCoV RNA real-time reverse transcription polymerase chain reaction (RT-PCR) test.

RT-PCR tests were performed at the Chinese Center for Disease Control and Prevention, targeting the coronavirus membrane gene (Lancet, 2020 Feb 15, 395-10223:497-506).

The COVID-19 patients recruited were 18-65 years old. When the patient's condition still worsened after being treated by basic therapy, MSC transplantation was recommended. When the patient was diagnosed with any type of cancers, or the doctor determined the condition to be very serious, then the patient was excluded from this study. Patients who participated in other clinical trials within 3 months were excluded.

This study was approved by the ethics committee. The safety and efficacy data of MSC treatment in patients were evaluated 14 days after MSC infusion.

### 2. Classification criteria of patients:

COVID-19 was clinically classified into four types:
1) Mild: Those with mild clinical symptoms and no imaging manifestation of pneumonia.
2) Normal: Those with fever, respiratory tract symptoms and imaging manifestation of pneumonia.
3) Severe: Those having any one of the following:
   respiratory distress with RR ≥ 30 times/minute;
   fingertip oxygen saturation in the resting state ≤ 93%;
   arterial partial pressure of oxygen (PaO₂)/oxygen inhalation concentration (FiO₂) ≤ 300 mmHg (1 mmHg = 0.133 kPa).
4) Critical: Those having one of the following:
   respiratory failure and requiring mechanical ventilation;
   occurrence of shock;
   combination with organ failure and requiring ICU monitoring and management.

**Table 1. Basic information for the 58 patients**

| **Item** | | **MSC group** | **Placebo group** | **P value** | **95% confidence interval** |
|---|---|---|---|---|---|
| **Number of enrollment** | | 29 | 29 | 1.000 | |

| **Gender** | | | | | |
|---|---|---|---|---|---|
| | **Male** | 12 (41.4) | 10 (34.5) | 0.7871 | |
| | **Female** | 17 (58.6) | 19 (65.5) | -- | |

| **Age** | | | | | |
|---|---|---|---|---|---|
| | **Median age** | 64 (54.5, 68) | 66 (59.5, 69.5) | 0.2221 | -7.418 to 1.763 |
| | **>50 (number, %)** | 24 (82.8) | 28 (96.6) | 0.194 | |
| | **30∼50 (number, %)** | 5 (17.2) | 1(3.4) | 0.194 | |

| **Clinical classification of COVID-19** | | | | | |
|---|---|---|---|---|---|
| | **Normal/mild** | 15 (51.7) | 16 (55.2) | 1.000 | |
| | **Severe** | 11 (37.9) | 10 (34.5) | 1.000 | |
| | **Critical** | 3 (10.3) | 3 (10.3) | 1.000 | |

| **Underlying medical conditions** | | | | | |
|---|---|---|---|---|---|
| | **Coronary heart disease** | 3 (10.3) | 3 (10.3) | 1.000 | |
| | **Diabetes** | 4 (13.8) | 4 (13.8) | 1.000 | |
| | **Brain diseases** | 3 (10.3) | 2 (6.9) | 1.000 | |
| | **Hypertension** | 12 (41.4) | 11 (37.9) | 1.000 | |
| | **Chronic lung diseases** | 1 (3.4) | 0 (0) | 1.000 | |
| | **Liver and kidney diseases** | 2 (6.9) | 3 (10.3) | 1.000 | |

| **Initial symptoms** | | | | | |
|---|---|---|---|---|---|
| | **Cough** | 22 (75.9) | 21 (72.4) | 1.000 | |
| | **Fever** | 16 (55.2) | 20 (69.0) | 0.417 | |
| | **Shortness of breath** | 17 (58.6) | 16 (55.2) | 0.730 | |
| | **Chest tightness** | 11 (37.9) | 14 (48.3) | 0.596 | |
| | **Fatigue** | 21 (72.4) | 19 (65.5) | 1.000 | |
| | **Muscle aches** | 9 (31.0) | 5 (17.2) | 0.358 | |
| | **Loss of appetite** | 3 (10.3) | 5 (17.2) | 0.787 | |
| | **Diarrhea** | 3 (10.3) | 3 (10.3) | 1.000 | |
| | **Vertigo** | 2 (6.9) | 5 (17.2) | 0.423 | |
| | **Nausea and vomiting** | 3 (10.3) | 3 (10.3) | 1.000 | |

| **Duration of symptoms before enrollment** | | | | | |
|---|---|---|---|---|---|
| | **Days** | 13 (9.5, 15.5) | 11 (8, 14.5) | 0.6908 | -2.628 to 3.939 |

| **Laboratory tests at enrollment** | | | | | |
|---|---|---|---|---|---|
| | **Total bilirubin (µmol/L)** | 11.1 (9.16, 15.2) | 10.9 (9.05, 13.8) | 0.6355 | -3.722 to 2.292 |
| | **C-reactive protein (mg/L)** | 51.4 (18.3,100.6) | 55.2 (32.0, 110.2) | 0.2648 | -14.14 to 50.33 |
| | **Procalcitonin (ng/mL)** | 0.10 (0.04, 0.14) | 0.09 (0.04, 0.17) | 0.2887 | -0.1216 to 0.4002 |
| | **Total white blood cell counts (/µl)** | 6.31 (4.20, 7.37) | 6.75 (4.92, 8.64) | 0.0781 | -4.211 to 0.2313 |
| | **Neutrophils (/µl)** | 5.66 (3.40, 7.48) | 4.34 (2.91, 5.95) | 0.0938 | -3.142 to 0.2534 |
| | **Lymphocytes (/µl)** | 0.64 (0.42, 1.12) | 0.93 (0.54, 1.24) | 0.1827 | -0.08833 to 0.4521 |
| | **Monocytes (/µl)** | 0.25 (0.19, 0.48) | 0.30 (0.20, 0.44) | 0.5397 | -0.2566 to 0.4848 |
| | **Hemoglobin (g/L)** | 130 (114, 145) | 126 (119, 136) | 0.8587 | -8.995 to 7.521 |
| | **Platelets (/µl)** | 162 (143-238) | 208 (158,254) | 0.4755 | -26.03 to 55.1 |
| | **Alanine transaminase (U/L)** | 40.0 (29.5-63.4) | 32.5 (23.2, 47.8) | 0.8469 | -26.15 to 21.54 |
| | **Aspartate aminotransferase (U/L)** | 31.9 (27.3-47.5) | 33.4 (23.5, 47.3) | 0.7854 | -20.64 to 15.69 |
| | **Creatinine (mg/dl)** | 62.9 (47.0-80.5) | 61.8 (53.1, 81.4) | 0.1683 | -118.6 to 21.23 |
| | **Serum potassium (mmol/L)** | 3.73 (3.46-3.88) | 3.79 (3.55, 4.13) | 0.2792 | -0.1147 to 0.3897 |
| | **Serum sodium (mmol/L)** | 139 (137-141) | 139 (135-141) | 0.8151 | -2.961 to 2.339 |
| | **Activated partial thromboplastin time (s)** | 27.5 (25.0-31.7) | 28.9 (26.2-32.2) | 0.5698 | -1.655 to 2.975 |
| | **Fibrinogen (g/L)** | 4.60 (3.42-4.98) | 4.59 (3.94-5.17) | 0.522 | -0.4026 to 0.7837 |

| | | | | | |
|---|---|---|---|---|---|
| Data in the table are presented as median (IQR) or percentage n (%). | | | | | |

### Example 2. MSCs of the present disclosure

Clinical grade ACE2-negative MSCs were provided free of charge by Shanghai University, Qingdao Haiwatson Biotechnology Group Co., Ltd. and the Institute of Basic Medical Sciences, Chinese Academy of Medical Sciences. This cell product was certificated by the State Food and Drug Administration (certification No. 2004L04792, 2006L01037 and CXSB 1900004).

The cell concentration was determined by using an automated cell counter. The sample volume was calculated based on the optimal cell sampling concentration and the number of target captures. If the concentration was too high, the volume of the suspension was adjusted to obtain an appropriate concentration and the cells were counted again.

### Example 3. ACE2-negative MSC treatment improved prognosis for COVID-19 patients, reduced NET, and promoted the production of SARS-CoV-2-specific antibodies

1. A randomized, single-blinded, placebo-controlled phase II trial was conducted in this example to evaluate the safety and efficacy of transplantation of ACE2-negative MSCs.

Fifty-eight COVID-19 patients (22 males and 36 females) were enrolled, wherein 31 patients with normal disease, 21 patients with severe disease and 6 patients with critical disease. They were randomly grouped into MSC-treated group or placebo group (29 patients in each group) at a ratio of 1:1. There was no difference in baseline characteristics between the two groups (Table 1). There was also no difference in other treatments received before and after transplantation between the MSC group and placebo group (Table 2).

All patients gave informed consent. Before intravenous infusion, MSCs were suspended in 100 ml of normal saline and the total number of cells was calculated at 1×10⁶ cells/kilogram of body weight. The window period for cell transplantation referred to the time when symptoms or/and signs were still worsening while the expected treatment was being performed. The infusion lasted about 40 minutes.

There were no significant adverse effects in both groups at and within 24 hours after MSC infusion.

**Table 2. Treatment received by patients in both groups before and after enrollment**

| **Item** | **Baseline** | | | **After treatment** | | |
|---|---|---|---|---|---|---|
| | MSC group | Placebo group | P value | MSC group | Placebo group | P value |
| **Combination therapy** | | | | | | |
| **Oxygen inhalation** | 27 (93.1) | 24 (88.9) | 0.423 | 26 (89.7) | 27 (93.1) | 1.000 |
| **Non-invasive mechanical ventilation** | 3 (10.3) | 2 (6.9) | 1.000 | 3 (10.3) | 3 (10.3) | 1.000 |
| **Invasive mechanical ventilation** | 0 | 0 | - | 0 | 2 (6.9) | 0.491 |

| **Steroid hormones** | | | | | | |
|---|---|---|---|---|---|---|
| **Number of patients** | 20 (70.0) | 19 (65.5) | 1.000 | 16 (55.2) | 17 (58.6) | 1.000 |
| **Days of dosing** | 4 (3, 6) | 4 (2, 7) | 0.7525 | 4 (1, 9) | 7 (5, 14) | 0.2294 |
| **Average daily dose** | 40 (40, 73.3) | 40 (40, 80) | 0.6547 | 24.4 (3, 41.7) | 28.6 (13.3, 46.4) | 0.7685 |

| **Antibiotics** | | | | | | |
|---|---|---|---|---|---|---|
| **Number of patients** | 18 (62.1) | 19 (65.5) | 1.000 | 16 (55.2) | 18 (62.1) | 0.790 |
| **Moxifloxacin** | 12 (41.4) | 18 (62.1) | 0.189 | 8 (27.6) | 16 (55.2) | 0.061 |
| **Days of dosing** | 4 (3.25, 6.75) | 3 (1.25, 4.75) | 0.1287 | 7 (0.5, 8.75) | 8 (2.25, 10) | 0.4577 |
| **Piperacillin and tazobactam** | 10 (34.5) | 8 (27.6) | 0.777 | 8 (27.6) | 5 (17.2) | 0.530 |
| **Days of dosing** | 6.5 (5, 9.75) | 2.5 (1.25, 16.25) | 0.8948 | 5 (0, 10.75) | 3.5 (0, 7.75) | 0.5512 |
| **Levofloxacin** | 4 (13.8) | 3 (10.3) | 1.000 | 3 (10.3) | 2 (6.9) | 1.000 |
| **Days of dosing** | 3 (1,5) | 3 (0, 11.5) | 0.833 | 3 (0, 6) | 0 (0, 7.5) | - |

| **Antiviral treatment** | | | | | | |
|---|---|---|---|---|---|---|
| **Number of patients** | 13 (44.8) | 17 (58.6) | 0.431 | 12 (41.4) | 15 (51.7) | 0.599 |
| **α-interferon** | 5 (17.2) | 9 (31.0) | 0.358 | 5 (17.2) | 9 (31.0) | 0.358 |
| **Days of dosing** | 5 (3, 10) | 5 (3, 7.25) | 0.5285 | 13 (10.5, 16.5) | 10.5 (5.5, 15) | 0.2938 |
| **Ribavirin** | 11 (37.9) | 12 (41.4) | 1.000 | 9 (31.0) | 13 (44.8) | 0.417 |
| **Days of dosing** | 4.5 (1.5, 5.75) | 2 (2, 4.5) | 0.3917 | 5 (0.25, 7.25) | 5 (5, 12) | 0.0725 |
| **Ganciclovir** | 9 (31.0) | 7 (24.1) | 0.770 | 1 (3.4) | 1 (3.4) | 1.000 |
| **Days of dosing** | 4 (3, 7) | 2 (1, 15.5) | 0.899 | - | - | - |

Data in the table are presented as median (IQR) or percentage n (%).

2. At the primary endpoint, the median hospitalization time of the MSC group (11 days, interquartile range 8-14 days) was shorter than that of the placebo group (15 days, interquartile range 11-19 days) (p = 0.0198) (Table 3). In addition, the median time for symptom relief in the MSC group (7 days, interquartile range 7-12 days) was also shorter than that of the placebo group (13 days, interquartile range 8-16 days) (p = 0.0194).

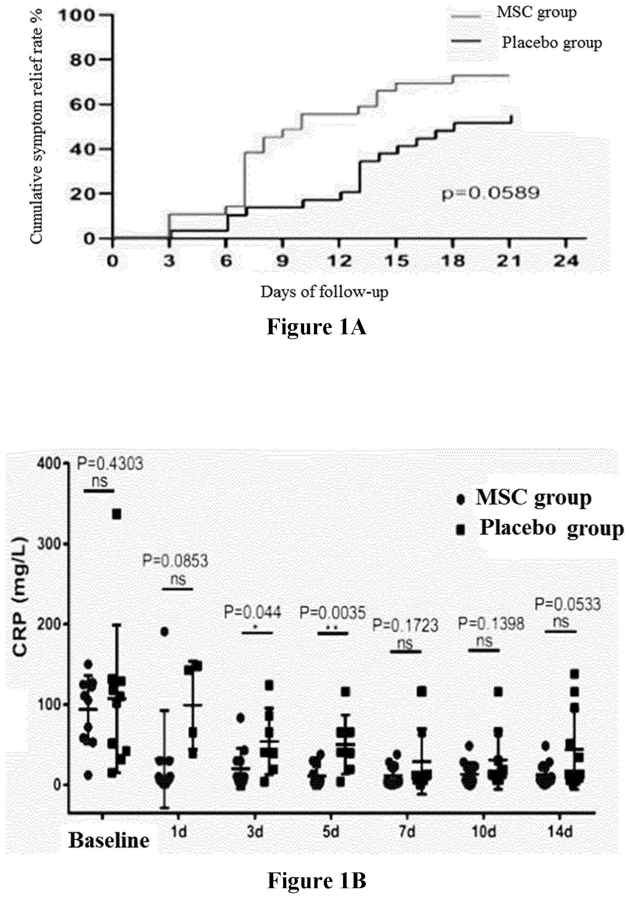

The improvement of symptoms of the patients in the MSC group on Day 7, Day 14 and Day 21 was better than that of the placebo group (p = 0.031, p = 0.0466, p = 0.0187) (Table 3). The cumulative symptom relief rate in the MSC group was higher than that of the placebo group (log-rank rank sum test p = 0.0589; hazard ratio 1.806; 95% confidence interval 0.9405 to 3.469) (Figure 1A). It was noticed that patients with severe or critical disease in the MSC-treated group had faster relief of symptom on Day 14 (p = 0.0405) and Day 21 (chi-square test p = 0.0157) than those in the placebo group (data not shown).

In addition, follow-up chest CT showed significant improvement (p = 0.0099, chi-square test) of diffuse lung density of both lungs in patients with severe or critical COVID-19 in the MSC group compared with that in the placebo group on Day 7 (p = 0.0099) and Day 21 (p = 0.0084) (Table 3). These results suggest that mesenchymal stem cells can significantly improve symptoms in patients with severe or critical disease.

**Table 3. Comparison of efficacy between patients in the MSC group and placebo group after treatment**

| **Item** | | **MSC group** | **Placebo group** | **P value¹** |
|---|---|---|---|---|
| **Improvement of clinical symptoms** | | | | |
| Day 7 | | | | 0.031 |
| | Symptoms vanishing* | 11 (37.9) | 4 (13.8) | |
| | Improvement of symptoms | 17 (58.6) | 19 (65.5) | |
| | No change | 1 (3.4) | 6 (20.7) | |
| Day 14 | | | | 0.0466 |
| | Symptoms vanishing | 19 (65.5) | 12 (41.4) | |
| | Improvement of symptoms | 9 (31.0) | 10 (34.5) | |
| | No change | 1 (3.4) | 7 (24.1) | |
| Day 21 | | | | 0.0187 |
| | Symptoms vanishing | 21 (72.4) | 16 (55.2) | |
| | Improvement of symptoms | 8 (27.6) | 6 (20.7) | |
| | No change | 0 | 7 (24.1) | |

| **Improvement of chest imaging** | | | | |
|---|---|---|---|---|
| Patients with normal/mild disease | | | | |
| Day 7 | | | | 0.5756 |
| | Improvement | 6 (20.7) | 7 (24.1) | |
| | No progression | 8 (27.6) | 9 (31.0) | |
| | Progression | 1 (3.4) | 0 | |
| Day 14 | | | | 0.3171 |
| | Improvement | 6 (20.7) | 7 (24.1) | |
| | No progression | 7 (24.1) | 9 (31.0) | |
| | Progression | 2 (6.9) | 0 | |
| Day 21 | | | | 0.5436 |
| | Improvement | 7 (24.1) | 7 (24.1) | |
| | No progression | 7 (24.1) | 9 (31.0) | |
| | Progression | 1 (3.4) | 0 | |

| Patients with severe/critical disease | | | | |
|---|---|---|---|---|
| Day 7 | | | | 0.0099 |
| | Improvement | 10 (34.5) | 2 (6.7) | |
| | No progression | 4 (13.8) | 9 (31.0) | |
| | Progression | 0 | 2 (6.7) | |
| Day 14 | | | | 0.0754 |
| | Improvement | 9 (31.0) | 3 (10.3) | |
| | No progression | 4 (13.8) | 6 (20.7) | |
| | Progression | 1 (3.4) | 4 (13.8) | |
| Day 21 | | | | 0.0084 |
| | Improvement | 11 (37.9) | 3 (10.3) | |
| | No progression | 3 (10.3) | 6 (20.7) | |
| | Progression | **0** | 4 (13.8) | |

| **Days required for symptoms vanishing**** | | | | |
|---|---|---|---|---|
| | | 7 (7, 12) | 13 (8, 16) | 0.0194^{#} |

| **Days of hospitalization**** | | | | |
|---|---|---|---|---|
| | | 11 (8, 14) | 15 (11, 19) | 0.0198^{#} |

| | | | | |
|---|---|---|---|---|
| 1, chi-square test; #, t-test. *, this item included the number of patients whose symptoms vanished and who were discharged from the hospital. **, assessed 21 days after treatment. Data in the table are presented as median (IQR) or percentage n (%). | | | | |

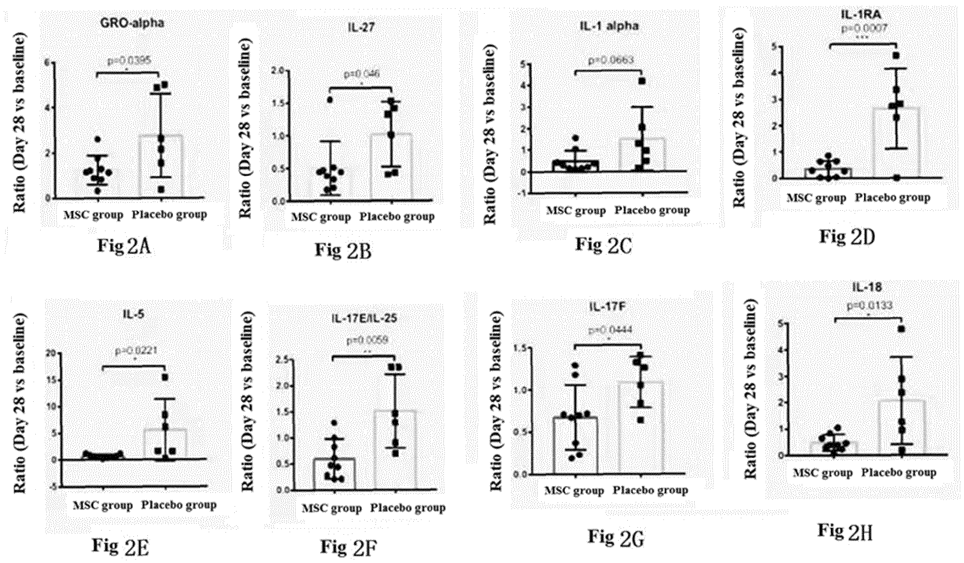

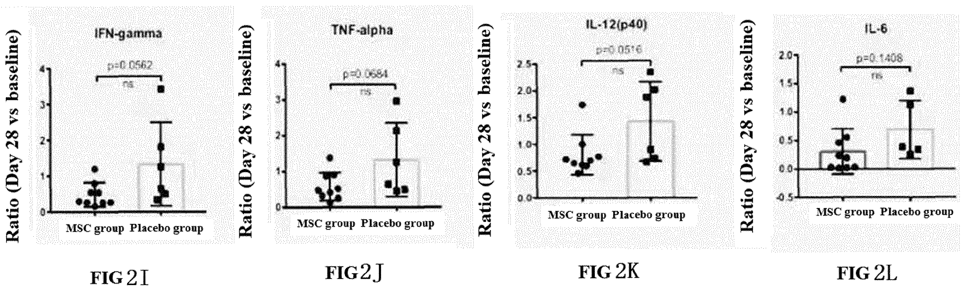

3. For the secondary endpoint, levels of serum C-reactive protein (CRP) in both groups were assessed to determine whether MSC infusion could modulate the immune system. CRP levels in patients with severe disease in the MSC group were significantly reduced compared to those of the placebo group, especially on Day 3 (20.27±7.604 mg/L versus 54.21±15.53 mg/L, p = 0.044) and on Day 5 (10.82±3.982 mg/L versus 50.16±13.87 mg/L, p = 0.0035) (Figure 1B). Plasma levels of pro-inflammatory cytokines (including IL-1RA, IL-18, IL-27, IL-17E, IL-25, IL-17F, GRO-alpha (CXCL-1) and IL-5) were significantly reduced in patients receiving MSC treatment on Day 28 (p < 0.05) (Figure 2A to Figure 2L). The 28-day mortality rate was 0.0% in the MSC group but 6.9% in the placebo group (Table 3).

4. Safety was assessed by monitoring vital signs 24 hours before and after treatment with MSC or placebo. Body temperature, pulse, respiratory rate, and systolic and diastolic blood pressure were similar between the two groups (Table 4). Serious adverse events were more serious in the placebo group compared to the MSC group, but the difference was not statistically significant. Common adverse effects were mild or moderate in severity (Table 5).

**Table 4. Assessment of vital signs of patients in the MSC group and placebo group**

| **Item** | **Baseline** | | | **After treatment** | | |
|---|---|---|---|---|---|---|
| | **MSC group** | **Placebo group** | **P value** | **MSC group** | **Placebo group** | **P value** |
| **Body temperature (°C)** | 36.7 (36.5, 38.0) | 36.6 (36.4, 36.8) | 0.2625 | 36.5 (36.3, 36.6) | 36.6 (36.4, 36.8) | 0.0137 |
| **Pulse (times/min)** | 78 (75.0, 86) | 80 (77, 90) | 0.4701 | 77 (71, 80) | 78 (75, 85) | 0.3846 |
| **Respiratory rate (times/min)** | 20 (18, 21) | 20 (19, 20) | 0.9326 | 20 (18, 22) | 20 (18, 20) | 0.5586 |
| **Systolic blood pressure (mmHg)** | 130 (118, 136) | 128 (119, 137) | 0.7764 | 130 (121, 135) | 130 (122, 135) | 0.9433 |
| **Diastolic blood pressure (mmHg)** | 79 (75, 81) | 74 (70, 80) | 0.1491 | 78 (75, 80) | 75 (70, 78) | 0.0407 |

| | | | | | | |
|---|---|---|---|---|---|---|
| Data in the table are presented as median (IQR). | | | | | | |

**Table 5. Records of adverse events in patients in the MSC group and placebo group**

| **Item** | **MSC group** | **Placebo group** |
|---|---|---|
| **Adverse events** | | |
| Cases | 3 (10.3) | 13 (44.8) |
| Consciousness disorder | 0 | 2 (6.9) |
| Urinary tract infection | 0 | 1 (3.4) |
| Headache | 0 | 1 (3.4) |
| Palpitation | 1 (3.4%) | 3 (10.3) |
| Fever | 0 | 3 (10.3) |
| Diarrhea/abdominal distension | 0 | 2 (6.9) |
| Loss of appetite | 0 | 1 (3.4) |
| Increased blood pressure | 1 (3.4) | 2 (6.9) |
| Somatalgia | 1 (3.4) | 3 (10.3) |

| **Laboratory tests within 3 days after administration** | | |
|---|---|---|
| Alanine aminotransferase | 12 (41.4) | 11 (37.9) |
| Hyperbilirubinemia | 2 (6.9) | 4 (13.8) |
| Elevated creatinine | 3 (10.3) | 2 (6.9) |

| **Number of deaths in 28 days** | | |
|---|---|---|
| | 0 | 2 (6.9) |

| | | |
|---|---|---|
| Data in the table are presented as percentage n (%). | | |

5. NET is an indicator of pathogenic immunothrombosis in COVID-19 patients (Manne et al., 2020). Thus, plasma NET-DNA levels before and after MSC treatment was compared by using Sytox green analysis.

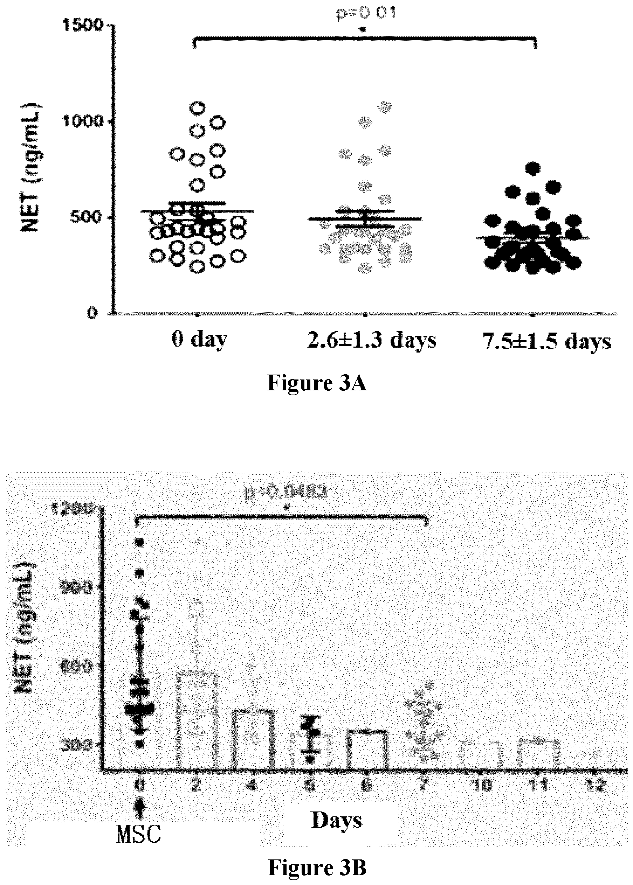

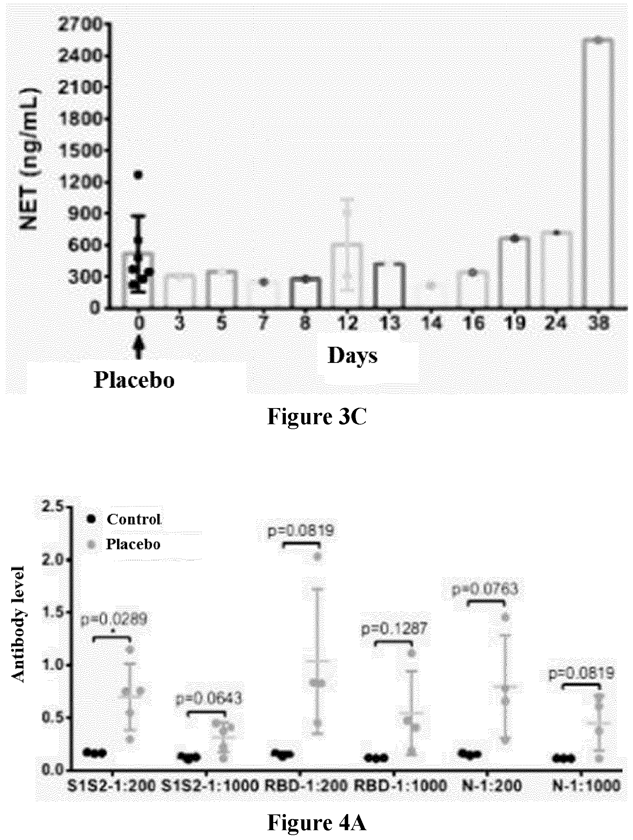

Plasma NET-DNA levels in MSC-treated patients slightly decreased on Day 2.6 compared to those before treatment (493.5±40.92 ng/mL versus 531.9±42.84 ng/mL). Plasma NET-DNA decreased on Day 7.5 after MSC treatment (395.91±24.93 ng/mL versus 531.89±42.83 ng/mL, p = 0.01) (Figure 3A). In addition, plasma NET-DNA levels in MSC-treated patients steadily decreased over time (Day 7 versus Day 0, p = 0.048) (Figure 3B). Almost no effect was observed in the placebo group (Figure 3C). These results suggest that MSC treatment can effectively reduce NET levels in the plasma of COVID-19 patients.

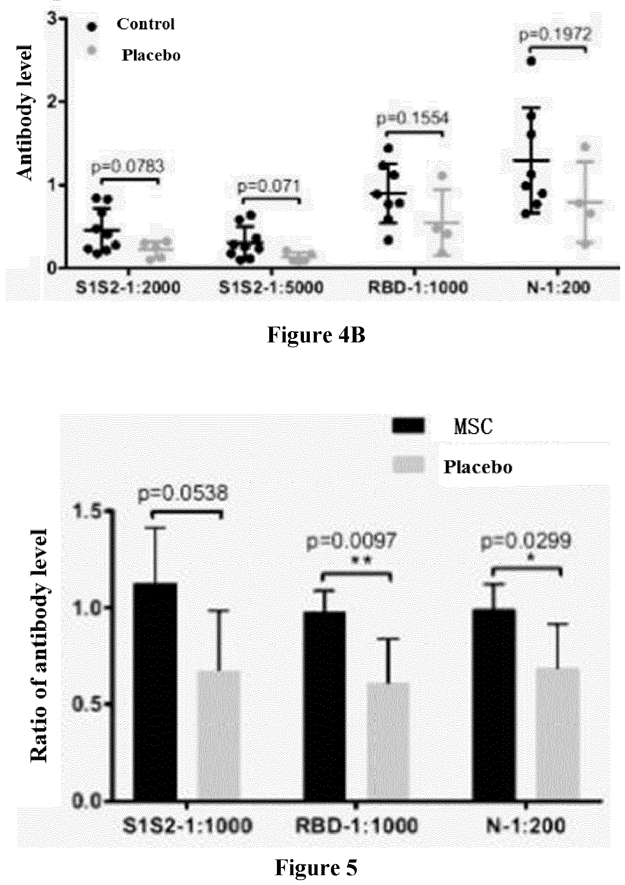

6. On Day 14 and Day 28 of MSC treatment, human plasma antibodies against SARS-CoV-2 spike S1 + S2 extracellular domain, against spike receptor binding domain, and against nucleocapsid were detected. On Day 28, plasma levels of antibodies against SARS-CoV-2 were slightly higher in the placebo group compared to healthy controls (no COVID-19 diagnosis) (Figure 4A). The plasma levels of SARS-CoV-2 antibodies in MSC-treated patients were significantly higher than in the placebo group (Figure 4B). In addition, the ratio of antibody levels between Day 28 and Day 14 in the MSC-treated group was approximately 1.0, higher than the ratio of approximately 0.5 in the placebo group (Figure 5).

These results suggest that MSC treatment not only improves clinical efficacy in COVID-19 patients, but also reduces levels of CRP, cytokines and NETs, and promotes the production of SARS-CoV-2-specific antibodies which lasts for longer time than that in placebo treatment.

7. C-reactive protein (CRP) is an indicator of systemic infection and inflammatory states. According to the exemplary test results from one patient (Table 6), the CRP level decreased from 105.50 ng/ml to 10.10 ng/ml. This indicates the relief of systemic infection and inflammation.

8. In addition, after MSC transplantation, the indicators for liver disorder, kidney injury and heart injury gradually returned to normal, which indicates that MSCs promote tissue repair after transplantation. In addition, chest CT scans of patients showed a reduction in pneumonia infiltration after MSC transplantation.

Clinical studies have shown that ACE2-negative MSCs have the potential of treating COVID-19 by suppressing the inflammatory response as well as promoting tissue repair. The study shows the potential of MSCs in the treatment of viral infections.

**Table 6. Exemplary results in patients with severe disease**

| | **Reference interval** | **Jan. 24** | **Jan. 30** | **Jan. 31** | **Feb. 1** | **Feb. 2** | **Feb. 4.** | **Feb. 6** | **Feb. 10** | **Feb. 13** |
|---|---|---|---|---|---|---|---|---|---|---|
| **C reactive protein (ng/ml)** | < 3.00 | 2.20 | 105.50 | NA | 191.00 | 83.40 | 13.60 | 22.70 | 18.30 | 10.10 |
| **Lymphocyte counts (×10⁹/L)** | 1.10-3.20 | 0.94 | 0.60 | 0.35 | 0.23 | 0.35 | 0.58 | 0.87 | 0.73 | 0.93 |
| **Leukocyte counts (×10⁹/L)** | 3.50-9.50 | 4.91 | 6.35 | 7.90 | 7.08 | 12.16 | 12.57 | 11.26 | 10.65 | 8.90 |
| **Neutrophil counts (×10⁹/L)** | 1.80-6.30 | 3.43 | 5.43 | 7.28 | 6.63 | 11.33 | 11.10 | 9.43 | 9.18 | 7.08 |
| **Monocyte counts (×10⁹/L)** | 0.10-0.60 | 0.38 | 0.25 | 0.17 | 0.13 | 0.35 | 0.61 | 0.52 | 0.48 | 0.56 |
| **Erythrocyte counts (×10¹²/L)** | 4.30-5.80 | 4.69 | 4.68 | 4.66 | 4.78 | 4.73 | 4.75 | 5.16 | 4.69 | 4.53 |
| **Hemoglobin (g/L)** | 130.00-175.00 | 145.00 | 147.00 | 145.00 | 146.00 | 142.00 | 145.00 | 155.00 | 145.00 | 137.00 |
| **Platelet counts (×10⁹/L)** | 125.00-350.00 | 153,00 | 148.00 | 169.00 | 230.00 | 271.00 | 268.00 | 279.00 | 332.00 | 279.00 |
| **Eosinophil counts (×10⁹/L)** | 0.02-0.52 | 0.02 | 0.02 | 0.02 | 0.02 | 0.02 | 0.05 | 0.15 | 0.14 | 0.14 |
| **Basophil counts (×10⁹/L** | 0.00-0.06 | 0.02 | 0.01 | 0.02 | 0.02 | 0.02 | 0.06 | 0.10 | 0.03 | 0.04 |
| **Total bilirubin (µmol/L)** | 5.00-21.00 | 7.00 | 23.00 | 21.70 | 19.80 | 14.20 | 15.80 | 16.50 | 12.50 | 8.70 |
| **Albumin (g/L)** | 40.00-55.00 | 41.70 | 32.30 | 29.70 | 29.90 | 31.60 | 33.00 | 32.20 | 30.10 | 29.10 |
| AST (U/L) | 15.00-40.00 | 14.00 | 33.00 | 48.00 | 57.00 | 39.00 | 34.00 | 23.00 | 25.00 | 19.00 |
| **Fibrinogen (g/L)** | 2.00-4.00 | 2.44 | 4.24 | NA | NA | 4.73 | NA | 3.12 | 3.84 | 3.73 |
| **Procalcitonin (ng/ml)** | < 0.10 | 0.11 | 0.12 | NA | NA | NA | 0.10 | 0.18 | 0.15 | < 0.10 |
| **Creatine kinase isoenzymes (ng/ml)** | < 3.60 | 0.90 | 0.12 | NA | 5.67 | 4.24 | NA | 0.88 | 0.90 | 0.61 |
| **Creatine kinase (U/L)** | 50.00.310.00 | 168.00 | 231.00 | NA | 513.00 | 316.00 | NA | 47.0.0 | 83.00 | 40.00 |
| **Glomerular filtration rate (ml/min)** | > 90.00 | 81.30 | 68.00 | 89.60 | 99.00 | 104.00 | 92.50 | 108.10 | 97.10 | 94.10 |
| **Potassium (mmol/L)** | 3.50.5.30 | 3.61 | 2.74 | 3.00 | 3.42 | 3.47 | 4.18 | 4.36 | 4.69 | 4.61 |
| **Sodium (mmol/L)** | 137.00.147.00 | 138.50 | 132.60 | 129.50 | 132.80 | 136.90 | 135.80 | 133.80 | 134.10 | 137.70 |
| **Myoglobin (ng/ml)** | 16.00.96.00 | 53.00 | 80.00 | NA | 138.00 | 77.00 | NA | 62.00 | 60.00 | 43.00 |
| **Troponin (ng/ml)** | < 0.056 | 0.10 | 0.07 | NA | 0.05 | 0.05 | NA | 0.02 | 0.04 | 0.04 |

### Example 4. ACE2-negative MSCs upregulated integrin signaling by upregulating the expression of kindlin-3 in immune cells and mitigated NET release and DVT in COVID-19 patients

Integrins play a key role in the immune response by mediating leukocyte adhesion and migration to the site of infection (Hynes, 2002). Members of the β2-integrin family are specifically expressed in leukocytes and bind to their ligands in an activation-dependent manner (Springer and Dustin, 2012).

After an immune challenge, activation of β2-integrin in leukocytes is induced by two key integrin activators, talin protein and kindlin-3 (Moser et al., 2009). Although the talin protein is extensively expressed, kindlin-3 is mainly expressed in hematopoietic cells. Loss or dysfunction of kindlin-3 in humans leads to insufficient leukocyte adhesion, manifested by recurrent infections and severe bleeding problems (Kuijpers et al., 2009; Malinin et al., 2009; Svensson et al., 2009; Xu et al., 2015; Xu et al., 2014).

In order to evaluate the effect of ACE2-negative MSC treatment on integrin signaling in the immune cells of COVID-19 patients, this example compared the expression levels of key integrin signaling molecules in PBMCs in MSC-treated or control COVID-19 patients.

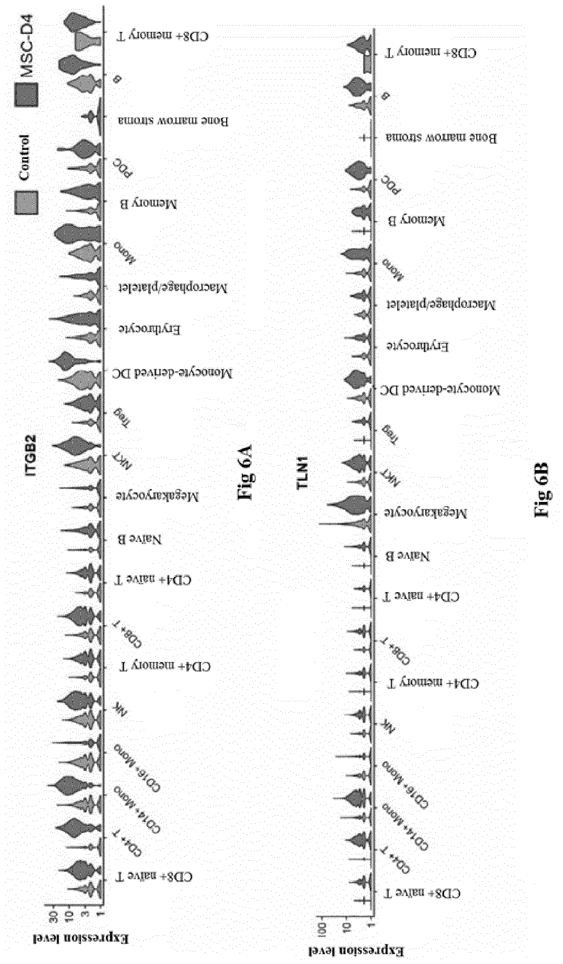

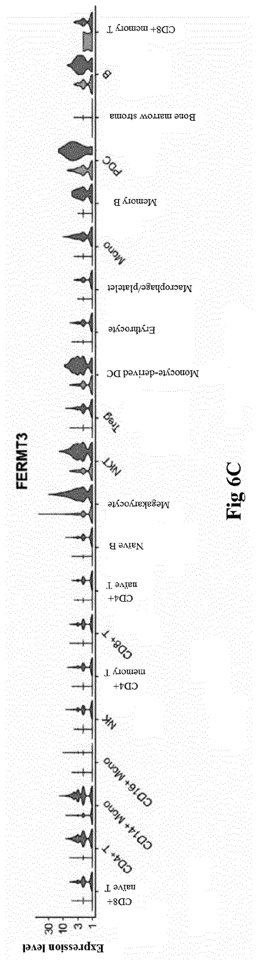

As shown in Figure 6A to Figure 6C, differential expression analysis of single-cell RNA sequencing data showed that gene expression of integrin β2 subunit, talin and kindlin-3 in innate immune cells and lymphocytes in MSC-treated patients was upregulated compared to that in untreated patients. These findings suggest that MSCs can promote the antiviral immune response in COVID-19 patients by promoting integrin-mediated adhesion and migration of immune cells.

Reports have shown that increased NETs are often observed at late stages in patients with COVID-19 acute respiratory distress syndrome (Barnes et al., 2020). NETs can promote thrombotic complications, especially DVT (Kimball et al., 2016; Laridan et al., 2019; Schonrich and Raftery, 2016; von Bruhl et al., 2012). Thrombosis is commonly observed in COVID-19 patients (Artifoni et al., 2020; Llitjos et al., 2020; Lodigiani et al., 2020). Recently, a new function of kindlin-3 that negatively regulates NET release and inhibits DVT in mice has been discovered in bone marrow cells (Xu et al., 2018; Yan et al., 2019).

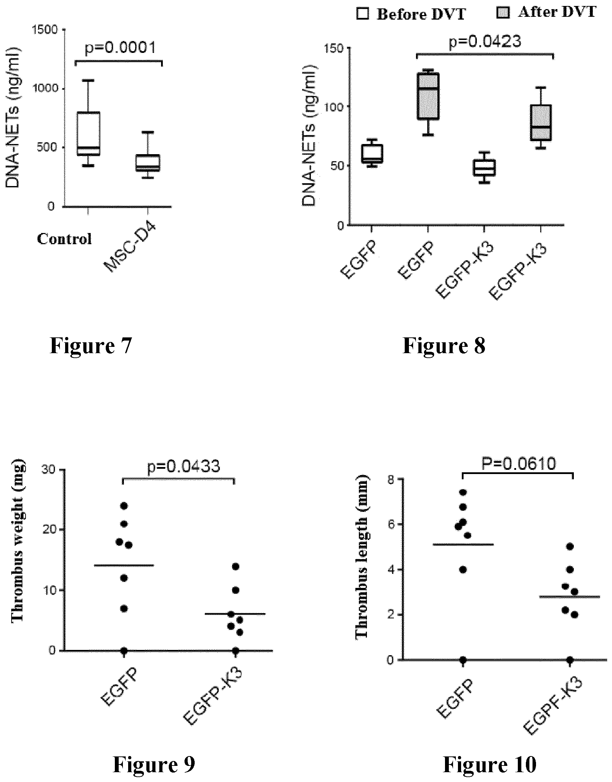

In this example, the discovery of upregulation of kindlin-3 in innate immune cells in MSC-treated COVID-19 patients motivated the inventors to study the NETs in the plasma of these patients. As shown in Figure 7, soluble DNA levels in the plasma of MSC-treated COVID-19 patients were significantly lower than that in untreated patients, indicating that MSC treatment could effectively inhibit NET release in COVID-19 patients. In addition, MSC-treated COVID-19 patients had a reduced risk of thrombotic complications.

### Example 5. Mouse model overexpressing kindlin-3

To illustrate the potential of kindlin-3 upregulation in cells to inhibit NET release and DVT in COVID-19 patients, the inventors further employed a mouse model with exogenous expression of EGFP-fused kindlin-3 in bone marrow cells.

Sca1⁺ bone marrow cells were isolated from wild-type C57BL/6 mice by using Sca1⁺ Selection Kit (Stemcell) and cultured in DMEM supplemented with 15% FBS, 20 ng/ml of IL-3, 50 ng/ml of IL-6, and 50 ng/ml of SCF. A lentiviral vector pLeGo-G2 with kindlin-3 was constructed to generate lentiviral particles expressing EGFP-fused kindlin-3, and the vector was further used to transduce bone marrow cells (MOI = 5). Lentiviral particles carrying empty pLeGo-G2 vectors were used to express EGFP alone in bone marrow cells (as a control). Two days after transduction, EGFP-positive cells were screened and transplanted into wild-type C57BL/6 recipient mice that received lethal irradiation. After eight weeks, these mice were analyzed. At the same time, as previously described by the inventors (Xu et al., 2018), the expression of EGFP-kindlin-3 and EGFP in the bone marrow cells of these mice was also evaluated by western blotting.

To trigger DVT in these mice, part of the inferior vena cava (IVC) was ligated to generate an inflammatory environment in the vena cava. As shown in Figure 8, IVC stenosis caused a significant increase in circulating DNA levels in the plasma of both types of mice, indicating that NET release was triggered.

Importantly, the inventors found that under the condition of IVC stenosis, the circulating DNA levels in mice expressing EGFP-kindlin-3 were significantly lower than those in mice expressing EGFP, demonstrating that upregulation of kindlin-3 in hematopoietic cells could effectively inhibit NET release in mice. Thrombosis was also significantly hindered in mice expressing EGFP-kindlin-3 compared to mice expressing EGFP alone (Figure 9 and Figure 10), which indicated that upregulation of kindlin-3 in hematopoietic cells also has the potential of inhibiting DVT, probably by inhibiting NET release.

### Discussions

MSC treatment upregulates integrin signaling in immune cells and may inhibit NET release in COVID-19 patients by upregulating the expression of kindlin-3 in immune cells. In conclusion, the inventors have revealed a stem cell MSC population with the potential of treating COVID-19 pneumonia. Further research shows that MSCs are susceptible to multiple signals and quickly adjusts their function in response to microenvironment. This makes MSCs to play multiple important roles in maintenance of homeostasis, modulation and reconstruction of immunity, tissue repair, and potentially in clinical treatment.

### References

Abdelwahab, S.F., Cocchi, F., Bagley, K.C., Kamin-Lewis, R., Gallo, R.C., DeVico, A., and Lewis, G.K. (2003). HIV-1-suppressive factors are secreted by CD4+ T cells during primary immune responses. Proc Natl Acad Sci U S A 100, 15006-15010.

Akiyama, M., Yamada, O., Hideshima, T., et al. (2004). TNFalpha induces rapid activation and nuclear translocation of telomerase in human lymphocytes. Biochem Biophys Res Commun. 316, 528-532.

Artifoni, M., Danic, G., Gautier, G., Gicquel, P., Boutoille, D., Raffi, F., Néel, A., and Lecomte, R. (2020). Systematic assessment of venous thromboembolism in COVID-19 patients receiving thromboprophylaxis: incidence and role of D-dimer as predictive factors. J Thromb Thrombolysis 50, 211-216.

Barnes, B., Adrover, J., Baxter-Stoltzfus, A., Borczuk, A., Cools-Lartigue, J., Crawford, J., Daβler-Plenker, J., Guerci, P., Huynh, C., Knight, J., et al. (2020). Targeting potential drivers of COVID-19: Neutrophil extracellular traps. J Exp Med. 217, e20200652.

Bendall, L., and Bradstock, K. (2014). G-CSF: From granulopoietic stimulant to bone marrow stem cell mobilizing agent. Cytokine Growth Factor Rev. 25, 355-367.

Benyelles, M., Episkopou, H., O'Donohue, M.F., et al. (2019). Impaired telomere integrity and rRNA biogenesis in PARN-deficient patients and knock-out models. EMBO Mol Med. 11, e10201.

Blauth, K., Zhang, X., Chopra, M., Rogan, S., and Markovic-Plese, S. (2015). The role of fractalkine (CX3CL1) in regulation of CD4(+) cell migration to the central nervous system in patients with relapsing-remitting multiple sclerosis. Clin Immunol 157, 121-132.

Brinkmann, V, Reichard, U., Goosmann, C., Fauler, B., Uhlemann, Y, Weiss, D., Weinrauch, Y, and Zychlinsky, A. (2004). Neutrophil extracellular traps kill bacteria.. Science 303, 1532-1535.

Bulati, M., Caruso, C., and Colonna-Romano, G. (2017). From lymphopoiesis to plasma cells differentiation, the age-related modifications of B cell compartment are influenced by "inflamm-ageing". Ageing Res Rev. 36, 125-136.

Cao, W., Bover, L., Cho, M., Wen, X., Hanabuchi, S., Bao, M., et al. (2009). Regulation of TLR7/9 responses in plasmacytoid dendritic cells by BST2 and ILT7 receptor interaction. J Exp Med. 206, 1603-1614.

Caplan, A.I. (1991). Mesenchymal stem cells. J Orthop Res. 9, 641-650.

Chen, G., Wu, D., Guo, W., Cao, Y, Huang, D., Wang, H., et al. (2020). Clinical and immunological features of severe and moderate coronavirus disease 2019. J Clin Invest. 130, 2620-2629.

Cisse, B., Caton, M.L., Lehner, M., Maeda, T., Scheu, S., Locksley, R., et al. (2008). Transcription factor E2-2 is an essential and specific regulator of plasmacytoid dendritic cell development. Cell 135, 37-48.

Daniel, C., Leppkes, M., Muñoz, L., Schley, G., Schett, G., and Herrmann, M. (2019). Extracellular DNA traps in inflammation, injury and healing. Nat Rev Nephrol 15, 559-575.

Ebner, S., Hofer, S., Nguyen, V.A., et al. (2002). A novel role for IL-3: human monocytes cultured in the presence of IL-3 and IL-4 differentiate into dendritic cells that produce less IL-12 and shift Th cell responses toward a Th2 cytokine pattern. J Immunol 168, 6199-6207.

Friedenstein, A.J., Chailakhyan, R.K., Latsinik, N.V, Panasyuk, A.F., and Keiliss-Borok, I.V (1974). Stromal cells responsible for transferring the microenvironment of the hemopoietic tissues. Cloning in vitro and retransplantation in vivo. Transplantation 17, 331-340.

Friedenstein AJ., Petrakova KV, Kurolesova AI., and GP., F. (1968). Heterotopic of bone marrow. Analysis of precursor cells for osteogenic and hematopoietic tissues. Transplantation 6, 230-247.

Ghosh, H.S., Cisse, B., Bunin, A., Lewis, K.L., and Reizis, B. (2010). Continuous expression of the transcription factor e2-2 maintains the cell fate of mature plasmacytoid dendritic cells. Immunity 33, 905-916.

Gilliet, M., Cao, W., and Liu, Y. (2008). Plasmacytoid dendritic cells: sensing nucleic acids in viral infection and autoimmune diseases. Nat Rev Immunol 8, 594-606.

Gómez, D., Diehl, M., Crosby, E., Weinkopff, T., and Debes, G. (2015). Effector T Cell Egress via Afferent Lymph Modulates Local Tissue Inflammation. J Immunol 195, 3531-3536.

Greenbaum, A.M., and Link, D.C. (2011). Mechanisms of G-CSF-mediated hematopoietic stem and progenitor mobilization. Leukemia 25, 211-217.

Honda, K., Ohba, Y, Yanai, H., Negishi, H., Mizutani, T., Takaoka, A., et al. (2005). Spatiotemporal regulation of MyD88-IRF-7 signalling for robust type-I interferon induction. Nature 434, 1035-1040.

Huang, C., Wang, Y, Li, X., Ren, L., Zhao, J., Hu, Y, et al. (2020). Lancet 395, 497-506.

Hynes, R.O. (2002). Integrins: bidirectional, allosteric signaling machines. Cell 110, 673-687.

Ivetic, A., Hoskins, G.H.L., and Hart, S.J. (2019). L-selectin: A Major Regulator of Leukocyte Adhesion, Migration and Signaling. Front Immunol 10, 1068.

Kang, R., Zhang, Q., Zeh, H., Lotze, M., and Tang, D. (2013). HMGB1 in cancer: good, bad, or both? . Clin Cancer Res. 19, 4046-4057.

Kato, M., Khan, S., d'Aniello, E., McDonald, K., and Hart, D. (2007). The novel endocytic and phagocytic C-Type lectin receptor DCL-1/CD302 on macrophages is colocalized with F-actin, suggesting a role in cell adhesion and migration. J Immunol 179, 6052-6063.

Kimball, A.S., Obi, A.T., Diaz, J.A., and Henke, P.K. (2016). The Emerging Role of NETs in Venous Thrombosis and Immunothrombosis. Front Immunol 7, 236.

Kuijpers, T.W., van de Vijver, E., Weterman, M.A., de Boer, M., Tool, A.T., van den Berg, T.K., Moser, M., Jakobs, M.E., Seeger, K., Sanal, O., et al. (2009). LAD-1/variant syndrome is caused by mutations in FERMT3. Blood 113, 4740-4746.

Kuzmina, Z., Krenn, K., Petkov, V, et al. (2013). CD19(+)CD21(low) B cells and patients at risk for NIH-defined chronic graft-versus-host disease with bronchiolitis obliterans syndrome. Blood 121, 1886-1895.

Laridan, E., Martinod, K., and De Meyer, S.F. (2019). Neutrophil Extracellular Traps in Arterial and Venous Thrombosis. Semin Thromb Hemost 45, 86-93.

Le Blanc, K., and Mougiakakos, D. (2012). Multipotent mesenchymal stromal cells and the innate immune system. Nat Rev Immunol 12, 383-396.

Leng, Z., Zhu, R., Hou, W., Feng, Y, Yang, Y, Han, Q., Shan, G., Meng, F., et al. (2020). Transplantation of ACE2- Mesenchymal Stem Cells Improves the Outcome of Patients with COVID-19 Pneumonia. Aging Dis. 11, 216-228.

Llitjos, J.F., Leclerc, M., Chochois, C., Monsallier, J.M., Ramakers, M., Auvray, M., and Merouani, K. (2020). High incidence of venous thromboembolic events in anticoagulated severe COVID-19 patients J Thromb Haemost. 10.1111.

Lodigiani, C., Iapichino, G., Carenzo, L., Cecconi, M., Ferrazzi, P., Sebastian, T., Kucher, N., Studt, J.D., Sacco, C., Alexia, B., et al. (2020). Venous and arterial thromboembolic complications in COVID-19 patients admitted to an academic hospital in Milan, Italy. Thromb Res. 191, 9-14.

Malinin, N.L., Zhang, L., Choi, J., Ciocea, A., Razorenova, O., Ma, YQ., Podrez, E.A., Tosi, M., Lennon, D.P., Caplan, A.I., et al. (2009). A point mutation in KINDLIN3 ablates activation of three integrin subfamilies in humans. Nat Med. 15, 313-318.

Mehta, P., Mcauley, D.F., Brown, M., et al. (2020). COVID-19: consider cytokine storm syndromes and immunosuppression. Lancet 395, 1033-1034.

Mojsilović, S., Jauković, A., Santibañez, J., and Bugarski, D. (2015). Interleukin-17 and its implication in the regulation of differentiation and function of hematopoietic and mesenchymal stem cells. Mediators Inflamm. 470458.

Mongini, P.K., Jackson, A.E., Tolani, S., et al. (2003). Role of complement-binding CD21/CD19/CD81 in enhancing human B cell protection from Fas-mediated apoptosis. J Immunol 171, 5244-5254.

Mori, S., Sakamoto, A., Yamashita, K., Fujimura, L., Arima, M., Hatano, M., Miyazaki, M., and Tokuhisa, T. (2004). Effect of c-fos overexpression on development and proliferation of peritoneal B cells. Int Immunol 16, 1477-1486.

Morrow, J.D., Chase, R.P., Parker, M.M., et al. (2019). RNA-sequencing across three matched tissues reveals shared and tissue-specific gene expression and pathway signatures of COPD. Respir Res. 20, 65.

Moser, M., Legate, K.R., Zent, R., and Fässler, R. (2009). The tail of integrins, talin, and kindlins. Science 324, 895-899.

Nagai, T., Devergne, O., Mueller, T.F., et al. (2003). Timing of IFN-beta exposure during human dendritic cell maturation and naive Th cell stimulation has contrasting effects on Th1 subset generation: a role for IFN-beta-mediated regulation of IL-12 family cytokines and IL-18 in naive Th cell differentiation. J Immunol 171, 5233-5243.

Nitschke, L., and Tsubata, T. (2004). Molecular interactions regulate BCR signal inhibition by CD22 and CD72. Trends Immunol 25, 543-550.

Okada, S., Wang, Z., Grigoriadis, A., Wagner, E., and von Rüden, T. (1994). Mice lacking c-fos have normal hematopoietic stem cells but exhibit altered B-cell differentiation due to an impaired bone marrow environment. Mol Cell Biol. 14, 382-390.

Pedrazza, L., Cunha, A.A., Luft, C., Nunes, N.K., Schimitz, F., Gassen, R.B., Breda, R.V, Donadio, M.V, de Souza Wyse, A.T., Pitrez, P.M.C., et al. (2017). Mesenchymal stem cells improves survival in LPS-induced acute lung injury acting through inhibition of NETs formation. J Cell Physiol 232, 3552-3564.

Pittenger, M.F., Mackay, A.M., Beck, S.C., Jaiswal, R.K., Douglas, R., Mosca, J.D., Moorman, M.A., Simonetti, D.W., Craig, S., and Marshak, D.R. (1999). Multilineage potential of adult human mesenchymal stem cells. Science 284, 143-147.

Prashad, S., Calvanese, V., Yao, C., Kaiser, J., Wang, Y, Sasidharan, R., Crooks, G., Magnusson, M., and Mikkola, H. (2015). GPI-80 defines self-renewal ability in hematopoietic stem cells during human development. Cell Stem Cell 16, 80-87.

Qiao, W., Wang, W., Laurenti, E., Turinsky, A., Wodak, S., Bader, G., Dick, J., and Zandstra, P. (2014). Intercellular network structure and regulatory motifs in the human hematopoietic system. Mol Syst Biol. 10, 741.

Ren, G., Zhao, X., Wang, Y, Zhang, X., Chen, X., Xu, C., Yuan, Z.R., Roberts, A.I., Zhang, L., Zheng, B., et al. (2012). CCR2-dependent recruitment of macrophages by tumor-educated mesenchymal stromal cells promotes tumor development and is mimicked by TNFalpha. Cell Stem Cell 11, 812-824.

Sandberg, K., Eloranta, M., Johannisson, A., and Alm, G. (1991). Flow cytometric analysis of natural interferon-alpha producing cells. Scand J Immunol 34, 565-576.

Schönrich, G., and Raftery, M.J. (2016). Neutrophil Extracellular Traps Go Viral. Front Immunol 7, 366.

Seifert, M., and Kuppers, R. (2016). Human memory B cells. Leukemia 30, 2283-2292.

Shi, C., Jia, T., Mendez-Ferrer, S., Hohl, T.M., Serbina, N.V, Lipuma, L., Leiner, I., Li, M.O., Frenette, P.S., and Pamer, E.G. (2011). Bone marrow mesenchymal stem and progenitor cells induce monocyte emigration in response to circulating toll-like receptor ligands. Immunity 34, 590-601.

Sinclair, A., Park, L., Shah, M., Drotar, M., Calaminus, S., Hopcroft, L., Kinstrie, R., Guitart, A., Dunn, K., Abraham, S., et al. (2016). CXCR2 and CXCL4 regulate survival and self-renewal of hematopoietic stem/progenitor cells. Blood 128, 371-383.

Spaggiari, G.M., Capobianco, A., Abdelrazik, H., Becchetti, F., Mingari, M.C., and Moretta, L. (2008). Mesenchymal stem cells inhibit natural killer-cell proliferation, cytotoxicity, and cytokine production: role of indoleamine 2,3-dioxygenase and prostaglandin E2. Blood 111, 1327-1333.

Springer, T.A., and Dustin, M.L. (2012). Integrin inside-out signaling and the immunological synapse. Curr Opin Cell Biol. 24, 107-115.

Svensson, L., Howarth, K., McDowall, A., Patzak, I., Evans, R., Ussar, S., Moser, M., Metin, A., Fried, M., Tomlinson, I., et al. (2009). Leukocyte adhesion deficiency-III is caused by mutations in KINDLIN3 affecting integrin activation.. Nat Med. 15, 306-312.

Takubo, K., Goda, N., Yamada, W., Iriuchishima, H., Ikeda, E., Kubota, Y, Shima, H., Johnson, R., Hirao, A., Suematsu, M., et al. (2010). Regulation of the HIF-1alpha level is essential for hematopoietic stem cells. Cell Stem Cell 7, 391-402.

Uccelli, A., Moretta, L., and Pistoia, V. (2008). Mesenchymal stem cells in health and disease. Nat Rev Immunol. 8, 726-736.

Venteicher, A.S., Abreu, E.B., Meng, Z., et al. (2009). A human telomerase holoenzyme protein required for Cajal body localization and telomere synthesis. Science 323, 644-648.

von Briihl, M.L., Stark, K., Steinhart, A., Chandraratne, S., Konrad, I., Lorenz, M., Khandoga, A., Tirniceriu, A., Coletti, R., Köllnberger, M., et al. (2012). Monocytes, neutrophils, and platelets cooperate to initiate and propagate venous thrombosis in mice in vivo. J Exp Med. 209, 819-835.

Wang, D., Hu, B., Hu, C., Zhu, F., Liu, X., Zhang, J., et al. (2020). JAMA 323, 1061-1069.

Weng, N.P., Hathcock, K.S., and Hodes, R.J. (1998). Regulation of telomere length and telomerase in T and B cells: a mechanism for maintaining replicative potential. Immunity 9, 151-157.

White, J.R., Lee, J.M., Young, P.R., Hertzberg, R.P., Jurewicz, A.J., Chaikin, M.A., Widdowson, K., Foley, J.J., Martin, L.D., Griswold, D.E., et al. (1998). Identification of a potent, selective non-peptide CXCR2 antagonist that inhibits interleukin-8-induced neutrophil migration. J Biol Chem. 273, 10095-10098.

Wu, Z., and McGoogan, J.M. (2020). Characteristics of and Important Lessons From the Coronavirus Disease 2019 (COVID-19) Outbreak in China: Summary of a Report of 72314 Cases From the Chinese Center for Disease Control and Prevention. JAMA 10.1001.

Xu, Z., Cai, J., Gao, J., White, G.C., Chen, F., and Ma, YQ. (2015). Interaction of kindlin-3 and β2-integrins differentially regulates neutrophil recruitment and NET release in mice. Blood 126, 373-377.

Xu, Z., Chen, X., Zhi, H., Gao, J., Bialkowska, K., Byzova, T.V, Pluskota, E., White, G., 2nd, , Liu, J., Plow, E.F., et al. (2014). Direct interaction of kindlin-3 with integrin αIIbβ3 in platelets is required for supporting arterial thrombosis in mice. Arterioscler Thromb Vasc Biol. 34, 1961-1967.

Xu, Z., Ni, B., Cao, Z., Zielonka, J., Gao, J., Chen, F., Kalyanaraman, B., White, G.C., and Ma, YQ. (2018). Kindlin-3 negatively regulates the release of neutrophil extracellular traps. J Leukoc Biol. 104, 597-602.

Yan, Y, Yang, H., Hu, X., Zhang, Z., Ge, S., Xu, Z., Gao, J., Liu, J., White, G.C., and Ma, YQ. (2019). Kindlin-3 in platelets and myeloid cells differentially regulates deep vein thrombosis in mice. Aging (Albany NY) 11, 6951-6959.

Zaccagnini, G., Gaetano, C., Della, P.L., et al. (2005). Telomerase Mediates Vascular Endothelial Growth Factor Dependent Responsiveness in a Rat Model of Hind Limb Ischemia. J Biol Chem. 280, 14790-14798.

Zhang, H., Li, H., Hillmer, E., Zhao, Y, Chrisikos, T., Hu, H., Wu, X., Thompson, E., Clise-Dwyer, K., Millerchip, K., et al. (2018). Genetic rescue of lineage-balanced blood cell production reveals a crucial role for STAT3 antiinflammatory activity in hematopoiesis. Proc Natl Acad Sci U S A 115, E2311-E2319.

Zhang, J., Grindley, J., Yin, T., Jayasinghe, S., He, X., Ross, J., Haug, J., Rupp, D., Porter-Westpfahl, K., Wiedemann, L., et al. (2006). PTEN maintains haematopoietic stem cells and acts in lineage choice and leukaemia prevention. Nature 441, 518-522.

Zhao, C., Matsushita, T., Ha, N.V, et al. (2020). CD22 and CD72 contribute to the development of scleroderma in a murine model. J Dermatol Sci. 97, 66-76.

## Claims

1. Use of mesenchymal stem cells in preparing a medicament, wherein:
the mesenchymal stem cells are ACE2-negative mesenchymal stem cells;
the medicament is used for treating thrombotic complications of viral pneumonia;
preferably, the medicament is used for any one selected from the following or a combination thereof: improving the symptoms of thrombotic complications of viral pneumonia, reducing the incidence of thrombotic complications of viral pneumonia, improving the prognosis of thrombotic complications of viral pneumonia, and inhibiting the release of neutrophil extracellular traps (NET).

2. The use according to claim 1, wherein:
the mesenchymal stem cells are selected from the group consisting of adipose-derived mesenchymal stem cells, umbilical cord blood mesenchymal stem cells, umbilical cord mesenchymal stem cells, bone marrow mesenchymal stem cells and placental mesenchymal stem cells;
the mesenchymal stem cells are autologous mesenchymal stem cells or allogeneic mesenchymal stem cells;
preferably, the mesenchymal stem cells are human mesenchymal stem cells.

3. The use according to claim 1, the virus is a coronavirus selected from the group consisting of SARS-CoV, MERS-CoV, 2019-nCoV and variants thereof.

4. The use according to claim 1, wherein the viral pneumonia is severe viral pneumonia or critical viral pneumonia.

5. The use according to claim 1, wherein:
before being administered to a patient, the mesenchymal stem cells are cultured under a condition selected from any one of the following or a combination thereof:
a temperature of 30°C to 40°C;
4% to 6% of CO₂;
DMEM/F12 medium supplemented with any one selected from the following or a combination thereof:
0.1% to 30% w/v of FBS,
1% to 3% w/v of antibiotics, and
0.1 mM to 30 mM of GlutaMAX^{™}-I.

6. A method of treating thrombotic complications of viral pneumonia, including a step of administering a therapeutically effective amount of mesenchymal stem cells to a patient, wherein:
the mesenchymal stem cells are used for any one selected from the following: improving the symptoms of thrombotic complications of viral pneumonia, reducing the incidence of thrombotic complications of viral pneumonia, improving the prognosis of thrombotic complications of viral pneumonia, and inhibiting the release of neutrophil extracellular traps;
the mesenchymal stem cells are selected from the group consisting of adipose-derived mesenchymal stem cells, umbilical cord blood mesenchymal stem cells, umbilical cord mesenchymal stem cells, bone marrow mesenchymal stem cells and placental mesenchymal stem cells;
the mesenchymal stem cells are autologous mesenchymal stem cells or allogeneic mesenchymal stem cells;
preferably, the mesenchymal stem cells are human mesenchymal stem cells;
the mesenchymal stem cells are ACE2-negative mesenchymal stem cells;
the virus is a coronavirus selected from the group consisting of SARS-CoV, MERS-CoV, 2019-nCoV and variants thereof.

7. The method according to claim 6, wherein the therapeutically effective amount is 0.1×10⁵ to 9×10⁶ mesenchymal stem cells per kilogram of body weight, preferably 1×10⁶ mesenchymal stem cells per kilogram of body weight.

8. The method according to claim 6, wherein the viral pneumonia is severe viral pneumonia or critical viral pneumonia.

9. The method according to claim 6, wherein before being administered to a patient, the mesenchymal stem cells are cultured under a condition selected from any one of the following or a combination thereof:
a temperature of 30°C to 40°C;
4% to 6% of CO₂;
DMEM/F12 medium supplemented with any one selected from the following or a combination thereof:
0.1% to 30% w/v of FBS,
1% to 3% w/v of antibiotics, and
0.1 mM to 30 mM of GlutaMAX^{™}-I.

10. Use of a NET release inhibitor in preparing a medicament, wherein:
the medicament is used for treating viral pneumonia;
preferably, the medicament is used for any one selected from the following: improving the symptoms of thrombotic complications of viral pneumonia, reducing the incidence of thrombotic complications of viral pneumonia, and improving the prognosis of thrombotic complications of viral pneumonia;
the NET release inhibitor is selected from the group consisting of kindlin-3, an agent that promotes the expression of kindlin-3, a cell that expresses kindlin-3 on its surface, and a viral vector that expresses kindlin-3.

11. The use according to claim 10, wherein:
the virus is a coronavirus selected from the group consisting of SARS-CoV, MERS-CoV, 2019-nCoV and variants thereof.

12. The use according to claim 10, wherein the viral pneumonia is severe viral pneumonia or critical viral pneumonia.

13. The use according to claim 10, the agent that promotes the expression of kindlin-3 is ACE2-negative mesenchymal stem cells;
the mesenchymal stem cells are selected from the group consisting of adipose-derived mesenchymal stem cells, umbilical cord blood mesenchymal stem cells, umbilical cord mesenchymal stem cells, bone marrow mesenchymal stem cells and placental mesenchymal stem cells;
the mesenchymal stem cells are autologous mesenchymal stem cells or allogeneic mesenchymal stem cells;
preferably, the mesenchymal stem cells are human mesenchymal stem cells.

14. A method of treating thrombotic complications of viral pneumonia, including a step of administering a therapeutically effective amount of a NET release inhibitor to a patient, wherein:
the NET release inhibitor is used for any one selected from the following: improving the symptoms of thrombotic complications of viral pneumonia, reducing the incidence of thrombotic complications of viral pneumonia, and improving the prognosis of thrombotic complications of viral pneumonia.
the NET release inhibitor is selected from the group consisting of kindlin-3, an agent that promotes the expression of kindlin-3, a cell that expresses kindlin-3 on its surface, and a viral vector that expresses kindlin-3.

15. The method according to claim 14, wherein:
the virus is a coronavirus selected from the group consisting of SARS-CoV, MERS-CoV, 2019-nCoV and variants thereof.

16. The method according to claim 14, wherein the viral pneumonia is severe viral pneumonia or critical viral pneumonia.
